# EUROPEAN PATENT APPLICATION

(11) **EP 2 572 746 A1**
(43) Date of publication of application: **27.03.2013**
(21) Application number: 11182631.9
(22) Date of filing: 23.09.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a safety needle device (1) comprising a needle hub (1.1) including a guide track (1.1.1), a needle (1.2) coupled to the needle hub (1.1), and a needle shield (1.3) telescopically coupled to the needle hub (1.1). The guide track (1.1.1) includes a first axial section (1.1.3), a second axial section (1.1.4), and a locking section (1.1.9). The needle (1.2) has a distal tip (1.2.1). The needle shield (1.3) includes a radial protrusion (1.3.1) adapted to engage the guide track (1.1.1). When the needle shield (1.3) is in a first axial position (PA1) and a first angular position (P1) relative to the needle hub (1.1), the radial protrusion (1.3.1) is in the first axial section (1.1.3) and the needle shield (1.3) covers the distal tip (1.2.1) of the needle (1.2). When the needle shield (1.3) is in a second axial position (PA2) and a second angular position (P2), the radial protrusion (1.3.1) is in the second axial section (1.1.4) and the distal tip (1.2.1) of the needle (1.2) is exposed from the needle shield (1.3). When the needle shield (1.3) is in a third axial position (PA3) and a third angular position (P3), the radial protrusion (1.3.1) is adjacent the locking section (1.1.9) and the needle shield (1.3) covers the distal tip (1.2.1) of the needle (1.2). The locking section (1.1.9) is adapted to engage the radial protrusion (1.3.1) to prevent movement of the needle shield (1.3) to the second axial position (PA2). A spring (1.5) applies an axially biasing force and a rotationally biasing force on the needle shield (1.3) relative to the needle hub (1.1). The axially biasing force biases the needle shield (1.3) toward the first axial position (PA1) and the third axial position (PA3), and the rotationally biasing force biases the needle shield (1.3) toward the third angular position (P3) relative to the needle hub (1.1).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a safety needle device comprises a needle hub including a guide track, a needle coupled to the needle hub, and a needle shield telescopically coupled to the needle hub. The guide track includes a first axial section, a second axial section, and a locking section. The needle has a distal tip. The needle shield includes a radial protrusion adapted to engage the guide track. When the needle shield is in a first axial position and a first angular position relative to the needle hub, the radial protrusion is in the first axial section and the needle shield covers the distal tip of the needle. When the needle shield is in a second axial position and a second angular position, the radial protrusion is in the second axial section and the distal tip of the needle is exposed from the needle shield. When the needle shield is in a third axial position and a third angular position, the radial protrusion is adjacent the locking section and the needle shield covers the distal tip of the needle. The locking section is adapted to engage the radial protrusion to prevent movement of the needle shield to the second axial position. A spring applies an axially biasing force and a rotationally biasing force on the needle shield relative to the needle hub. The axially biasing force biases the needle shield toward the first axial position and the third axial position, and the rotationally biasing force biases the needle shield toward the third angular position relative to the needle hub.

The needle hub may include a thread adapted to engage an injection device.

In an exemplary embodiment, the guide track includes an axial divider between the first axial section and the second axial section. The axial divider abuts the radial protrusion when the radial protrusion is in the first axial section of the guide track.

In an exemplary embodiment, the needle hub includes a stop tab formed in the guide track, the stop tab adapted to abut the radial protrusion to prevent separation of the radial protrusion from the guide track.

In an exemplary embodiment, the radial protrusion includes a proximal ramped side and a distal ramped side. The proximal ramped side is adapted to engage a first inclined section connecting the first axial section to the second axial section of the guide track and the distal ramped side is adapted to engage a second inclined section connecting the second axial section to the locking section of the guide track.

In an exemplary embodiment, the needle safety device further comprises a bearing element rotatably coupled to a distal face of the needle shield. The bearing element includes a collar adapted to mate with a first aperture in the needle shield. The collar includes a retaining tab adapted to engage the distal face of the needle shield. The bearing element includes a spacer adapted to abut the distal face of the needle shield.

In another exemplary embodiment, a safety needle device comprises a needle hub including a first guide track and a second guide track, a needle coupled to the needle hub, and a needle shield telescopically coupled to the needle hub. The second guide track includes a first axial section. The first guide track includes a second axial section and a locking section. The needle has a distal tip. The needle shield includes a first radial protrusion adapted to engage the first guide track and a second radial protrusion adapted to engage the second guide track. When the needle shield is in a first axial position and a first angular position relative to the needle hub, the second radial protrusion is in the first axial section and the needle shield covers the distal tip of the needle. When the needle shield is in a second axial position and a second angular position, the second radial protrusion is in the second axial section and the distal tip of the needle is exposed from the needle shield. When the needle shield is in a third axial position and a third angular position, the first radial protrusion is adjacent the locking section and the needle shield covers the distal tip of the needle, the locking section is adapted to engage the first radial protrusion to prevent movement of the needle shield to the second axial position. A spring applies an axially biasing force and a rotationally biasing force on the needle shield relative to the needle hub. The axially biasing force biases the needle shield toward the first axial position and the third axial position. The rotationally biasing force biases the needle shield toward the third angular position relative to the needle hub.

In an exemplary embodiment, the second guide track includes an axial divider adapted to abut the second radial protrusion when the second radial protrusion is in the first axial section of the second guide track. The needle hub includes a stop tab formed in the first guide track and/or the second guide track. The stop tab is adapted to abut the first radial protrusion and/or the second radial protrusion to prevent separation of the first radial protrusion from the first guide track and/or to prevent separation of the second radial protrusion from the second guide track. The first radial protrusion includes a proximal ramped side and the second radial protrusion includes a distal ramped side. The proximal ramped side is adapted to engage a first inclined section in the first guide track and the distal ramped side is adapted to engage a second inclined section in the second guide track.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an explosion view of an exemplary embodiment of a needle safety device;
- Figure 2: shows a sectional view of an exemplary embodiment of a needle safety device positioned in a first axial position;
- Figure 3: shows a schematic illustration of a guide track according to an exemplary embodiment of a needle safety device;
- Figures 4A and 4B: illustrate schematically a first and a second guide track according to an exemplary embodiment of a needle safety device;
- Figure 5: shows an isometric view of an exemplary embodiment of a needle hub of a needle safety device;
- Figure 6: shows an isometric view of an exemplary embodiment of a needle shield of a needle safety device;
- Figure 7: shows a sectional view of an exemplary embodiment of a needle safety device in a second axial position;

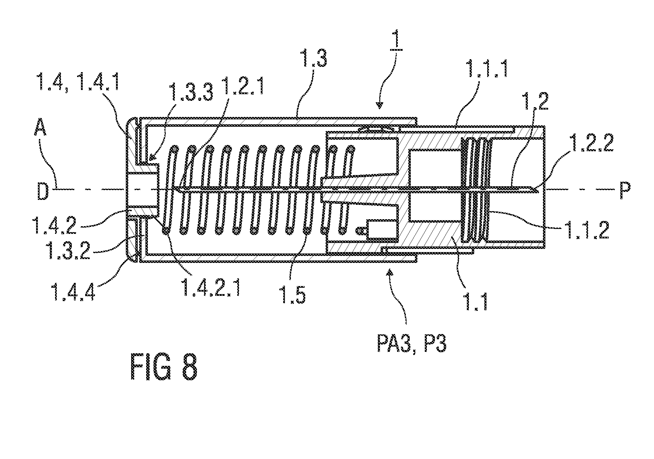

- Figure 8: shows a sectional view of an exemplary embodiment of a needle safety device in a third axial position;

Corresponding parts are marked with the same reference symbols in all figures.

### Detailed Description

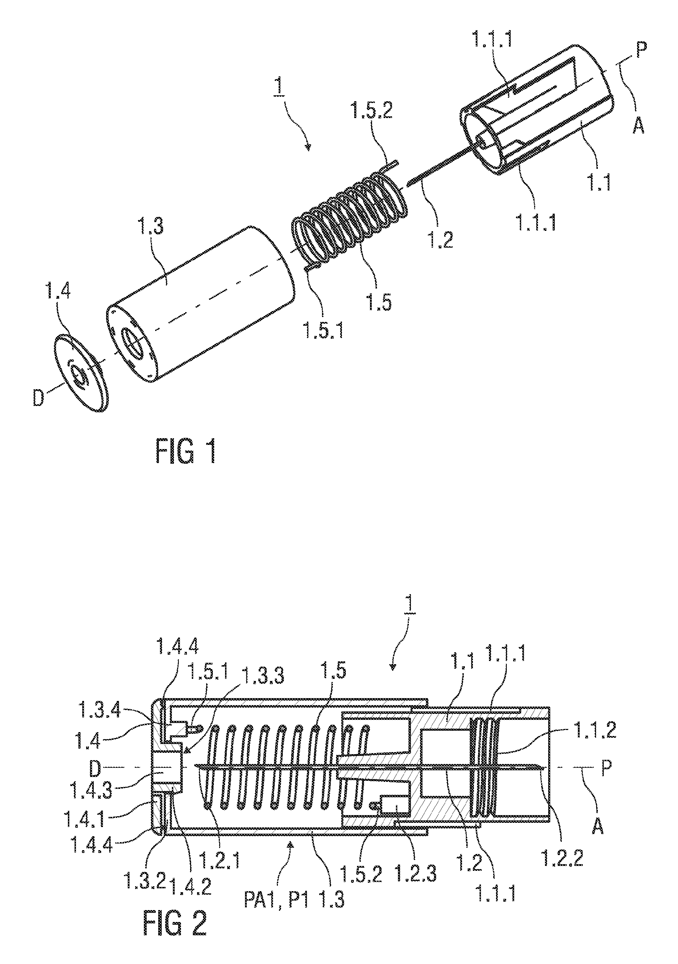

Figure 1 shows an exemplary embodiment of a needle safety device 1 according to the present invention. In an exemplary embodiment, the needle safety device 1 comprises a needle hub 1.1, a needle 1.2 coupled to the needle hub 1.1, and a needle shield 1.3 resiliently telescopically coupled to the needle hub 1.1 by a spring 1.5. A bearing element 1.4 may be coupled to a distal end of the needle shield 1.3. The needle hub 1.1 may be adapted to be removably coupled to an injection device (e.g., a syringe, a pen injector, an auto-injector, etc.). For example, the needle hub 1.1 may include a threaded coupling, snap fit, bayonet fit, friction fit, etc. to mate with the injection device. In another exemplary embodiment, the needle hub 1.1 may be formed integrally with the injection device.

The needle hub 1.1 may have a guide track 1.1.1 formed on its outer surface. As explained further below, the guide track 1.1.1 may limit movement of the needle shield 1.3 relative to the needle hub 1.1 during use of the needle safety device 1.

Figure 2 shows an exemplary embodiment of a needle safety device 1. In an exemplary embodiment, the needle hub 1.1 includes a thread 1.1.2 for mating with a corresponding thread (not shown) on an injection device (not shown). As noted above, any coupling mechanism (e.g., snap fit, bayonet fit, friction fit, etc.) may be utilized by the needle hub 1.1 to engage the injection device. The needle 1.2 is coupled to the needle hub 1.1 and has a proximal tip 1.2.2 adapted to pierce a septum of a container of medicament in the injection device and a distal tip 1.2.1 adapted to pierce an injection site. The needle 1.2 may be disposed along a longitudinal axis A of the needle safety device 1.

In an exemplary embodiment, the needle shield 1.3 is telescopically coupled to the needle hub 1.1. A distal end 1.5.1 of the spring 1.5 is coupled to a first retaining bush 1.3.4 on the needle shield 1.3, and a proximal end 1.5.2 of the spring 1.5 is coupled to a second retaining bush 1.2.3 on the needle hub 1.1. In an exemplary embodiment, the spring 1.5 is pre-loaded with a torque to impart a rotational biasing force on the needle shield 1.3 relative to the needle hub 1.1.

The needle shield 1.3 includes a first aperture 1.3.3 which allows the needle 1.2 to pass through when the needle shield 1.3 is pressed against an injection site.

The bearing element 1.4 is coupled to a distal face 1.3.2 of the needle shield 1.3. In an exemplary embodiment, the bearing element 1.4 includes a collar 1.4.2 which is adapted to engage the first aperture 1.3.3. The collar 1.4.2 includes a second aperture 1.4.3 which allows the needle 1.2 to pass through when the needle shield 1.3 is pressed against an injection site. The collar 1.4.2 also includes a retaining tab 1.4.2.1 adapted to engage the distal face 1.3.2 of the needle shield 1.3. The bearing element 1.4 may further include a spacer 1.4.4 adapted to provide an axial space between a proximal surface 1.4.1 of the bearing element 1.4 and the distal face 1.3.2 of the needle shield 1.3. In an exemplary embodiment, the bearing element 1.4 is capable of rotating (about the axis A) relative to the needle shield 1.3 by the engagement of the collar 1.4.2 in the first aperture 1.3.3. Rotation of the bearing element 1.4 relative to the needle shield 1.3 may be facilitated by the spacer 1.4.4 which limits frictional contact between the proximal surface 1.4.1 of the bearing element 14 and the distal face 1.3.2 of the needle shield 1.3.

In the exemplary embodiment shown in Figure 2, the needle shield 1.3 is in a first axial position (PA1) and a first angular position (P1) relative to the needle hub 1.1. In the first axial position (PA1), the needle shield 1.3 is in an extended position in which the distal tip 1.2.1 of the needle 1.2 is covered. The spring 1.5 imparts an axially biasing force on the needle shield 1.3 to bias the needle shield 1.3 in the first axial position (PA1). In the first angular position (P1), the needle shield 1.3 is subject to a rotational force from the spring 1.5.

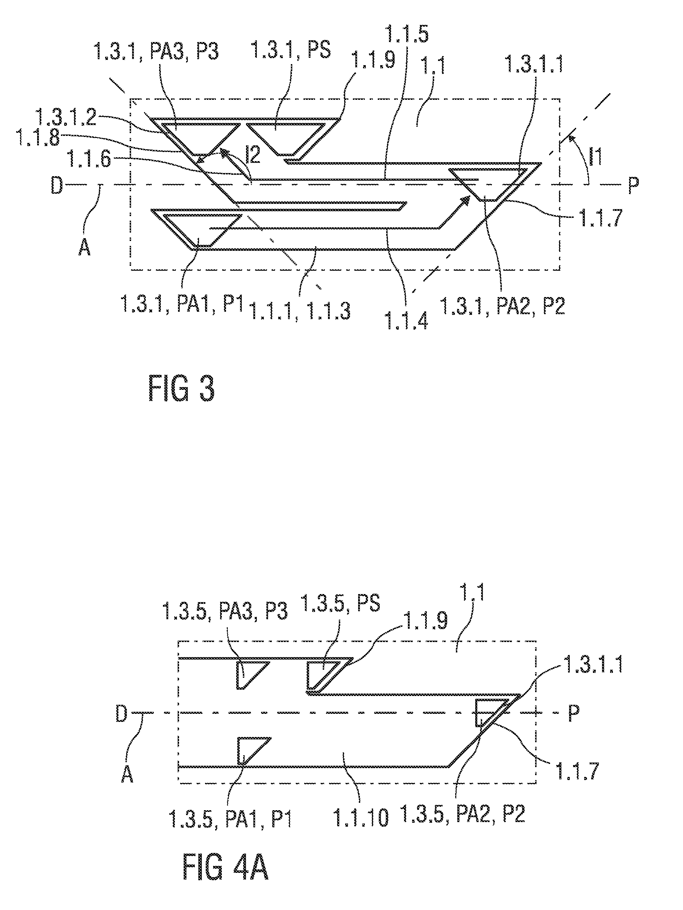

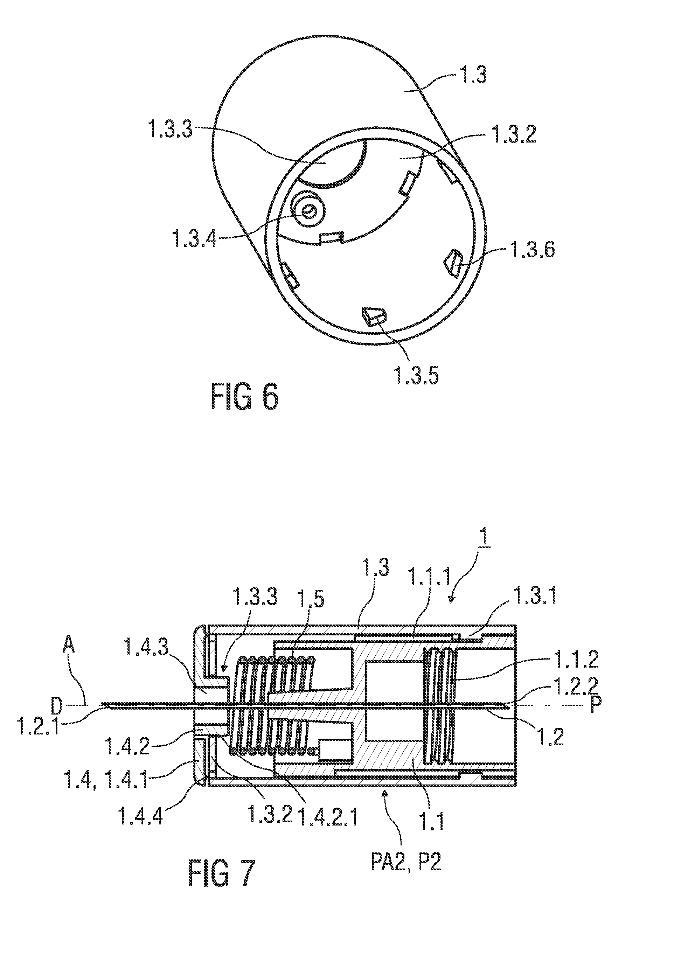

Figure 3 shows an exemplary embodiment of a coupling between the needle shield 1.3 and the needle hub 1.1. The needle shield 1.3 may include one or more radial protrusions 1.3.1 formed on an internal surface of the needle shield 1.3 which are adapted to engage one or more guide tracks 1.1.1 on the needle hub 1.1. Figure 6 shows an exemplary embodiment of the needle shield 1.3 including at least one radial protrusion 1.3.1.

Referring back to Figure 3, the radial protrusion 1.3.1 is engaged in the guide track 1.1.1. When the needle shield 1.3 is in the first angular position (P1), the radial protrusion 1.3.1 may be in a distal portion of a first axial section 1.1.3 of the guide track 1.1.1. The radial protrusion may be constrained in the first axial section 1.1.3 by an axial divider 1.1.13 formed on the needle hub 1.1.

Referring to Figure 7, when the needle safety device 1 is pressed against an injection site, the needle shield 1.3 is moved proximally relative to the needle hub 1.1 into a second axial position (PA2). In the second axial position (PA2), the distal tip 1.2.1 of the needle 1.2 is exposed.

Referring back to Figure 3, as the needle shield 1.3 is moved proximally, the spring 1.5 compresses and a proximal ramped surface of the radial protrusion 1.3.1 may abut a proximal bearing 1.1.7 of the guide track 1.1.1, which along with the rotational force of the spring 1.5, causes the radial protrusion 1.3.1 to move into a first inclined section 1.1.4 of the guide track 1.1.1. The needle sleeve 1.3 can rotate relative to the bearing element 1.4 so that no rotational force is transmitted to the injection site.

Figure 7 shows an exemplary embodiment of the needle shield 1.3 in a third axial position (PA3) after the needle safety device 1 is removed from the injection site. As the needle safety device 1 is removed from the injection site, the needle shield 1.3 moves distally relative to the needle hub 1.1 under force of the spring 1.5. In the third axial position (PA3), the distal tip 1.2.1 of the needle 1.2 is covered by the needle shield 1.3. As the needle shield 1.3 moves into the third axial position (PA3), the rotational force of the spring 1.5 causes the needle shield 1.3 to move into a third angular position (P3) relative to the needle hub 1.1.

Referring back to Figure 3, as the needle shield 1.3 is moved distally, the radial protrusion 1.3.1 moves distally in a second axial section 1.1.5 of the guide track 1.1.1. When the radial protrusion 1.3.1 reaches a distal end of the second axial section 1.1.5, the rotational force of the spring 1.5, causes the radial protrusion to move into a second inclined section 1.1.6 of the guide track 1.1.1. A distal ramped surface of the radial protrusion 1.3.1 may abut a distal bearing 1.1.8 to facilitate movement of the radial protrusion 1.3.1 into the second inclined section 1.1.6.

As shown in the exemplary embodiment in Figure 3, a locking section 1.9 of the guide track 1.1.1 may be formed adjacent the second inclined section 1.1.8. When the needle shield 1.3 is in the third axial position (PA3), attempts to move the needle shield 1.3 proximally relative to the needle hub 1.1 are prevented, because the radial protrusion 1.3.1 abuts the locking section 1.9 of the guide track 1.1.1. The radial protrusion 1.3.1 remains aligned with the locking section 1.9 due to rotational force of the spring 1.5. In the third axial position (PA3), the needle safety device 1 is in a needle-safe position (PS) and the distal tip 1.2.1 of the needle 1.2 cannot be exposed.

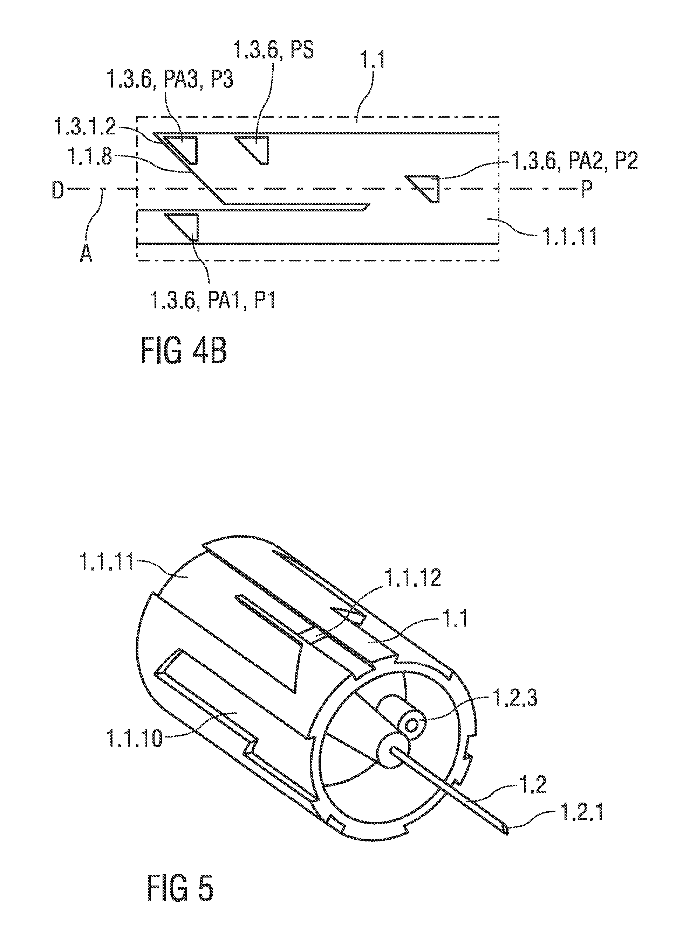

In another exemplary embodiment of a needle safety device 1 according to the present invention, function of components of the guide track 1.1.1 may be implemented in complementary tracks formed on the needle hub 1.1 which engage complementary radial protrusions 1.3.1 on the needle shield 1.3. Figures 4A, 4B and 5 show an exemplary embodiment of the needle safety device 1 utilizing complementary tracks.

Figure 4A shows an exemplary embodiment of a first guide track 1.1.10 which engages a first radial protrusion 1.3.5 on the needle shield 1.3. The first radial protrusion 1.3.5 includes a ramped proximal side 1.3.1.1. Figure 4B shows an exemplary embodiment of a second guide track 1.1.11 which engages a second radial protrusion 1.3.6 on the needle shield 1.3. The second radial protrusion 1.3.6 includes a ramped distal side 1.3.1.2.

As shown in Figure 4B, in the first axial position (PA1), the second radial protrusion 1.3.6 is maintained in the first axial section 1.1.3 by the axial divider 1.1.13, and thus the needle shield 1.3 remains in the first angular position (P1) relative to the needle hub 1.1. As shown in Figure 4A, in the first axial position (PA1), the first guide track 1.1.10 does not include an axial divider. Thus, in the first axial position (PA1), the abutment of the second radial protrusion 1.3.6 and the axial divider 1.1.13 maintains the needle shield 1.3 in the first angular position (P1) relative to the needle hub 1.1.

As shown in Figure 4B, in the second axial position (PA2), the second radial protrusion 1.3.6 has moved proximally beyond the axial divider 1.1.13, and the needle shield 1.3 rotates relative to the needle hub 1.1. As shown in Figure 4A, rotation of the needle shield 1.3 relative to the needle hub 1.1 is constrained by the first inclined section 1.1.4 formed in the first guide track 1.1.10. The first guide track 1.1.10 may include the proximal bearing 1.1.7 to engage the ramped proximal side 1.3.1.1 of the first radial protrusion 1.3.5 to facilitate movement from the first axial section 1.1.3 into the first inclined section 1.1.4.

As shown in Figure 4A, the first guide track 1.1.10 may include the second axial section 1.1.5. When the needle shield 1.3 moves distally relative to the needle hub 1.1 and the first radial protrusion 1.3.5 moves distally beyond the second axial section 1.1.5, the first guide track 1.1.10 does not constrain rotation of the needle shield 1.3 relative to the needle hub 1.1.

As shown in Figure 4B, in the third axial position (PA3), the second radial protrusion 1.3.6 has moved distally, and the ramped distal side 1.3.1.2 may engage the distal bearing 1.1.8 to facilitate movement of the second radial protrusion 1.3.6 from the second axial section 1.1.5 to the second inclined section 1.1.6. The second inclined section 1.1.6 may constrain rotation of the needle shield 1.3 relative to the needle hub 1.1 beyond the third angular position (P3).

Referring back to Figure 4A, the first guide track 1.3.10 may include the locking section 1.1.9 to engage the first radial protrusion 1.3.5 to prevent proximal movement of the needle shield 1.3 relative to the needle hub 1.1 when the needle shield 1.3 is in the third axial position (PA3). In this position, the needle safety device 1 is needle-safe and can prevent re-exposure of the distal tip 1.2.1 of the needle 1.2.

Figure 5 shows an exemplary embodiment of a needle hub 1.1 according to the present invention. The needle hub 1.1 may include one or more stop tabs 1.1.12 formed in the first guide track 1.1.10 and/or the second guide track 1.1.11 to prevent the first and/or second radial protrusions 1.3.5, 1.3.6 from exiting the guide tracks. The radial protrusions may abut the stop tabs when the needle shield is in the first, second and/or third axial positions.

A removable film may be disposed on a distal face of the bearing element 1.4 to maintain sterility of the needle 1.2.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A safety needle device (1) comprising:
a needle hub (1.1) including a guide track (1.1.1), the guide track (1.1.1) including a first axial section (1.1.3), a second axial section (1.1.4), and a locking section (1.1.9);
a needle (1.2) coupled to the needle hub (1.1), the needle (1.2) having a distal tip (1.2.1);
a needle shield (1.3) telescopically coupled to the needle hub (1.1), the needle shield (1.3) including a radial protrusion (1.3.1) adapted to engage the guide track (1.1.1),
wherein, when the needle shield (1.3) is in a first axial position (PA1) and a first angular position (P1) relative to the needle hub (1.1), the radial protrusion (1.3.1) is in the first axial section (1.1.3) and the needle shield (1.3) covers the distal tip (1.2.1) of the needle (1.2),
wherein, when the needle shield (1.3) is in a second axial position (PA2) and a second angular position (P2), the radial protrusion (1.3.1) is in the second axial section (1.1.4) and the distal tip (1.2.1) of the needle (1.2) is exposed from the needle shield (1.3), and
wherein, when the needle shield (1.3) is in a third axial position (PA3) and a third angular position (P3), the radial protrusion (1.3.1) is adjacent the locking section (1.1.9) and the needle shield (1.3) covers the distal tip (1.2.1) of the needle (1.2), the locking section (1.1.9) is adapted to engage the radial protrusion (1.3.1) to prevent movement of the needle shield (1.3) to the second axial position (PA2); and
a spring (1.5) applying an axially biasing force and a rotationally biasing force on the needle shield (1.3) relative to the needle hub (1.1), the axially biasing force biasing the needle shield (1.3) toward the first axial position (PA1) and the third axial position (PA3), the rotationally biasing force biasing the needle shield (1.3) toward the third angular position (P3) relative to the needle hub (1.1).

2. The needle safety device (1) according to claim 1, wherein the needle hub (1) includes a thread (1.1.2) adapted to engage an injection device.

3. The needle safety device (1) according to any of the preceding claims, wherein the guide track (1.1.1) includes an axial divider (1.1.13) between the first axial section (1.1.3) and the second axial section (1.1.4), wherein the axial divider (1.1.13) abuts the radial protrusion (1.3.1) when the radial protrusion (1.3.1) is in the first axial section (1.1.3) of the guide track (1.1.1).

4. The needle safety device (1) according to any of the preceding claims, wherein the needle hub (1.1) includes a stop tab (1.4.2.1) formed in the guide track (1.1.1), the stop tab (1.4.2.1) adapted to abut the radial protrusion (1.3.1) to prevent separation of the radial protrusion (1.3.1) from the guide track (1.1.1).

5. The needle safety device (1) according to any of the preceding claims, wherein the radial protrusion (1.3.1) includes a proximal ramped side (1.3.1.1) and a distal ramped side (1.3.1.2).

6. The needle safety device (1) according to claim 5, wherein the proximal ramped side (1.3.1.1) is adapted to engage a first inclined section (1.1.4) connecting the first axial section (1.1.3) to the second axial section (1.1.5) of the guide track (1.1.1) and the distal ramped side (1.3.1.2) is adapted to engage a second inclined section (1.1.6) connecting the second axial section (1.1.5) to the locking section (1.1.9) of the guide track (1.1.1).

7. The needle safety device (1) according to any of the preceding claims, further comprising:
a bearing element (1.4) rotatably coupled to a distal face (1.3.2) of the needle shield (1.3).

8. The needle safety device (1) according to claim 7, wherein the bearing element (1.4) includes a collar (1.4.2) adapted to mate with a first aperture (1.3.3) in the needle shield (1.3).

9. The needle safety device (1) according to claim 8, wherein the collar (1.4.2) includes a retaining tab (1.4.2.1) adapted to engage the distal face (1.3.2) of the needle shield (1.3).

10. The needle safety device (1) according to claim 7, wherein the bearing element (1.4) includes a spacer (1.4.4) adapted to abut the distal face (1.3.2) of the needle shield (1.3).

11. A safety needle device (1) comprising:
a needle hub (1.1) including a first guide track (1.1.10) and a second guide track (1.1.11), the second guide track (1.1.11) including a first axial section (1.1.3), the first guide track (1.1.10) including a second axial section (1.1.4) and a locking section (1.1.9);
a needle (1.2) coupled to the needle hub (1.1), the needle (1.2) having a distal tip (1.2.1);
a needle shield (1.3) telescopically coupled to the needle hub (1.1), the needle shield (1.3) including a first radial protrusion (1.3.5) adapted to engage the first guide track (1.1.10) and a second radial protrusion (1.3.6) adapted to engage the second guide track (1.1.11),
wherein, when the needle shield (1.3) is in a first axial position (PA1) and a first angular position (P1) relative to the needle hub (1.1), the second radial protrusion (1.3.6) is in the first axial section (1.1.3) and the needle shield (1.3) covers the distal tip (1.2.1) of the needle (1.2).
wherein, when the needle shield (1.3) is in a second axial position (PA2) and a second angular position (P2), the second radial protrusion (1.3.6) is in the second axial section (1.1.4) and the distal tip (1.2.1) of the needle (1.2) is exposed from the needle shield (1.3), and
wherein, when the needle shield (1.3) is in a third axial position (PA3) and a third angular position (P3), the first radial protrusion (1.3.5) is adjacent the locking section (1.1.9) and the needle shield (1.3) covers the distal tip (1.2.1) of the needle (1.2), the locking section (1.1.9) is adapted to engage the first radial protrusion (1.3.5) to prevent movement of the needle shield (1.3) to the second axial position (PA2); and
a spring (1.5) applying an axially biasing force and a rotationally biasing force on the needle shield (1.3) relative to the needle hub (1.1), the axially biasing force biasing the needle shield (1.3) toward the first axial position (PA1) and the third axial position (PA3), the rotationally biasing force biasing the needle shield (1.3) toward the third angular position (P3) relative to the needle hub (1.1).

12. The needle safety device (1) according to claim 11, wherein the second guide track (1.1.11) includes an axial divider (1.1.13) adapted to abut the second radial protrusion (1.3.6) when the second radial protrusion (1.3.6) is in the first axial section (1.1.3) of the second guide track (1.1.11).

13. The needle safety device (1) according to claims 11 or 12, wherein the needle hub (1.1) includes a stop tab (1.4.2.1) formed in the first guide track (1.1.10) and/or the second guide track (1.1.11), the stop tab (1.4.2.1) adapted to abut the first radial protrusion (1.3.5) and/or the second radial protrusion (1.3.6) to prevent separation of the first radial protrusion (1.3.5) from the first guide track (1.1.10) and/or to prevent separation of the second radial protrusion (1.3.6) from the second guide track (1.1.11).

14. The needle safety device (1) according to claims 11, 12 or 13, wherein the first radial protrusion (1.3.5) includes a proximal ramped side (1.3.1.1) and the second radial protrusion (1.3.6) includes a distal ramped side (1.3.1.2).

15. The needle safety device (1) according to claim 14, wherein the proximal ramped side (1.3.1.1) is adapted to engage a first inclined section (1.1.4) in the first guide track (1.1.10) and the distal ramped side (1.3.1.2) is adapted to engage a second inclined section (1.1.6) in the second guide track (1.1.11).
